# EUROPÄISCHE PATENTSCHRIFT

(11) **EP 0 664 995 B1**
(45) Veröffentlichungstag und Bekanntmachung des Hinweises auf die Patenterteilung: **29.04.1998**
(21) Anmeldenummer: 95100640.2
(22) Anmeldetag: 19.01.1995
(51) Int. Cl.: A61F 5/56, G08B 3/10

(54) **Vorrichtung zur Unterdrückung des Schnarchens**
Anti-snoring device
Dispositif anti-ronflement

(30) Priorität: 01.02.1994 CH 294/94
(43) Veröffentlichungstag der Anmeldung: 02.08.1995
(73) Patentinhaber: Balkanyi, Alexander, Dr. Med., CH-8038 Zürich (CH)
(72) Erfinder: Balkanyi, Alexander, Dr. Med., CH-8038 Zürich (CH)
(74) Vertreter: Blum, Rudolf Emil Ernst

(56) Entgegenhaltungen:
- EP-A- 0 145 160
- WO-A-90/11585
- DE-A- 3 719 074
- FR-A- 2 618 327

## Beschreibung

Die Erfindung betrifft eine Vorrichtung zur Unterdrückung des Schnarchens gemäss dem Oberbegriff der unabhängigen Ansprüche.

Es wurden verschiedene Vorrichtungen vorgeschlagen, die das Schnarchen unterdrücken sollen. Einige davon, wie z.B. jene, die in EP-A-145 160 beschrieben, und als Basis für die Oberbegriffe der unabhängigen Ansprüche 1 und 13 verwendet ist, senden bei Detektion eines Schnarchgeräusches ein akustisches Signal aus. Da sich der Schnarchende jedoch an dieses Signal gewöhnt, muss dessen Lautstärke und Dauer mit der Zeit erhöht werden. Dies geschieht in EP-A-145 160 dadurch, dass ein Zähler zum Zählen aufeinander folgender Schnarchsignale vorgesehen ist. Je höher der Zählerstand ist, desto lauter und/oder länger werden die Signale. Ein an das Gerät gewohnter Schnarcher benötigt sehr laute und starke Signale, bevor er mit dem Schnarchen aufhört. Dies kann zu Gehörschäden sowie einer unerwünschten Störung der Umgebung führen. Um dies zu vermeiden, muss die Lautstärke und Dauer der Signale limitiert sein. Es ist aber sehr schwierig, eine maximale Lautstärke zu finden, die einerseits garantiert, dass jeder Schnarcher darauf reagiert, und andererseits aber die Gefahr einer Gehörschädigung und/oder einer Störung der Umgebung ausschliesst.

Deshalb stellt sich die Aufgabe, eine Vorrichtung der eingangs erwähnten Art bereitzustellen, die die obigen Nachteile nicht aufweist. Insbesondere soll sie eine Gewöhnung des Schnarchers ausschliessen, ohne dass sie übermässig laute Signale verwenden muss.

Diese Aufgabe wird durch die Vorrichtung gemäss den unabhängigen Ansprüchen gelöst.

Indem nicht alle Signale die gleiche spektrale Zusammensetzung (d.h. das gleiche Fourier-Spektrum) haben, kann eine Gewöhnung praktisch ausgeschlossen werden. So können dem Schnarchenden verschieden hohe Töne vorgespielt werden. Vorzugsweise werden diese Töne bei andauerndem Schnarchen zunehmend unangenehm, indem z.B. spektrale Komponenten höherer Frequenz stärker werden.

Die spektrale Zusammensetzung der Signale kann auch nach einem Zufallsprinzip ausgewählt und/oder zeitlich variiert werden, was eine Gewöhnung weiter erschwert.

Vorzugsweise wird das Gerät in einem Stirnband integriert, welches ein Mikrofon trägt und mit einem Ohrhörer verbunden ist. Eine solche Vorrichtung ist bequem zu tragen und stört den Schlafenden nicht. Der Schallgeber kann jedoch auch im Stirnband selbst angeordnet sein und als Stirnlautsprecher den Schall direkt auf den Schädelknochen übertragen. In diesem Falle können alle Komponenten im Stirnband integriert werden, so dass die Vorrichtung den Schlafenden praktisch nicht behindert und störende Kabel entfallen.

Das Mikrofon kann aber z.B. auch ein Kehlkopfmikrofon sein.

Wird das Mikrofon am Körper getragen, so können die Schallwellen direkt über Knochen- bzw. Gewebeleitung übertragen und empfangen werden.

Weitere Vorteile der Erfindung ergeben sich aus der folgenden Beschreibung einiger bevorzugter Ausführungen anhand der Figuren. Dabei zeigen:
Figur 1 ein Blockschaltbild der Vorrichtung und
Figur 2 eine bevorzugte Anordnung des Geräts in einem Stirnband.

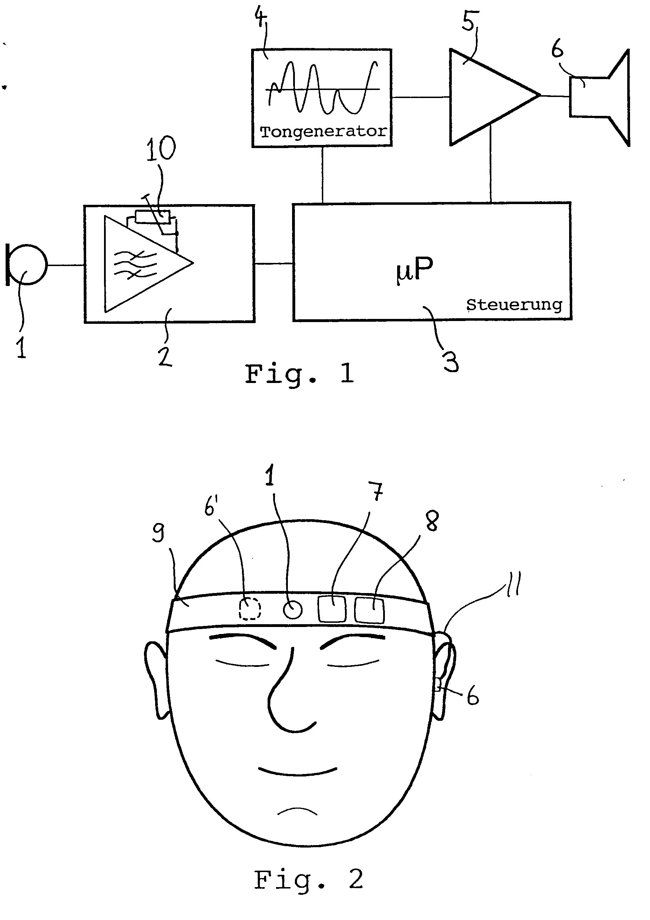

Figur 1 zeigt ein Blockschaltbild einer bevorzugten Ausführung der Erfindung. Zur Detektion des Schnarchens ist ein Mirkofon 1 vorgesehen, dessen Signal in einem kombinierten Verstärker, Filter und Demodulator 2 analysiert wird. Ein geeigneter Aufbau dieses Teils des Geräts wird z.B. in EP-A-145 160 und den darin zitierten Schriften beschrieben.

Das so erhaltene Signal wird der Steuerung 3 zugeführt. Bei der Steuerung handelt es sich vorzugsweise um einen geeignet programmierten Mikroprozessor.

Die Steuerung kontrolliert einen Tongenerator 4 und einen Verstärker 5. Beim Tongenerator 4 handelt es sich vorzugsweise um einen programmierbaren Baustein zur Erzeugung von Niederfrequenzsignalen, wie er dem Fachmann bekannt ist. Der Tongenerator kann jedoch auch Teil des Mikroprozessorsystems der Steuerung 3 sein, wobei der Mikroprozessor die Signale z.B. über einen Digital-Analogwandler direkt erzeugt. Ueber eine Steuerung des Verstärkers 5 kann die Lautstärke der Signale kontrolliert werden.

Das Ausgangssignal des Verstärkers 5 wird einem Schallgeber 6 zugeführt. Dabei handelt es sich vorzugsweise um ein elektrodynamisches oder piezoelektrisches Bauteil.

Das Gerät kann prinzipiell in verschiedenster Bauform hergestellt werden. So kann es zum Beipiel als Tischgerät ausgeführt sein. In diesem Fall sind alle Bauteile in einem Gehäuse integriert, welches sich z.B. auf dem Nachttisch des Schlafenden befindet.

Insbesondere wenn der Schnarchende jedoch nicht alleine schläft, empfiehlt es sich, das akustische Signal nicht auf dem Nachttisch sondern direkt beim Schnarchenden, z.B. an oder in dessen Ohr oder an seiner Stirn oder Schläfe, zu erzeugen. In einem solchen Fall kann z.B. die in EP-A-145 160 beschriebene Anordnung verwendet werden, in welcher das ganze Gerät kompakt aufgebaut und im Ohr des Schlafenden untergebracht wird. Es kann jedoch auch hinter dem Ohr angeordnet werden. Ein solches Gerät im oder hinter dem Ohr wird aber oftmals als unangenehm empfunden, insbesondere wenn der Schlafende auf dem betreffenden Ohr liegt. In diesem Falle kann auch die Schallaufnahme des Mikrofons beeinträchtigt werden.

Aus diesen Gründen wird eine Anordnung in einem Stirnband bevorzugt, wie sie in Figur 2 gezeigt wird. Bei diesem Stirnband 9 handelt es sich um ein weiches, elastisches Band, in welchem das Mikrofon 1, die elektronische Schaltung 7 und flache Batterien 8 untergebracht sind. Das Mikrofon berührt die Stirn oder Schläfe und nimmt die über den Schädeiknochen übertragenen Schnarchgeräusche auf. Der Schallgeber 6 ist im Ohr untergebracht und über ein kurzes Kabel 11 mit der Elektronik 7 verbunden.

Alternativ hierzu kann der Lautsprecher auch als Stirnlautsprecher 6' ausgeführt sein. In diesem Fall ist er im Stirnband 9 integriert und seine Membran wird direkt an die Stirn oder Schläfe des Schlafenden gedrückt. Die Schallübertragung erfolgt im wesentlichen über den Schädelknochen und ist für Dritte kaum hörbar. Bei der Ausführung mit Stirnlautsprecher 6' können der Ohrhörer 6 und das Zuleitungskabel 11 entfallen. Die ganze Vorrichtung ist nun also im Stirnband integriert, so dass ein maximaler Tragkomfort erreicht und bequemes Schlafen in allen Positionen möglich wird.

In einer weiteren bevorzugten Ausführung wird das Mikrofon mit einem Heftpflaster am Hals, vorzugsweise in der Nähe des Kehlkopfs befestigt und über ein Kabel mit der im Ohr angeordneten Elektronik verbunden.

Die Empfindlichkeit des Mikrofons kann über einen Regler 10 eingestellt werden. Sie ist so zu regeln, dass die Schnarchsignale detektiert werden, dass aber Umgebungsgeräusche (z.B. Schnarchen des Ehepartners) kein Signal auslösen.

Im Betrieb des Geräts wird ständig überwacht, ob vom Mikrofon 1 ein Schnarchen detektiert wird. Wird während eines vorgegebenen Zeitintervalls von z.B. einer Minute kein Schnarchen detektiert, so wird angenommen, dass der Schlafende nicht schnarcht. Sobald ein erstes Schnarchen wahrgenommen wird, wird ein erstes akustisches Signal (Signal 1) ausgegeben. Wird in einem Zeitintervall nach dem ersten Schnarchen ein weiteres Schnarchsignal wahrgenommen, so wird angenommen, dass der Schlafende zweimal hintereinander geschnarcht hat und es wird ein zweites akustisches Signal (Signal 2) ausgegeben. Folgt innerhalb des Zeitintervalls nach dem zweiten Schnarchen ein drittes, so wird ein drittes akustisches Signal ausgegeben (Singal 3), usw. Wenn innerhalb eines Zeitintervalls nach einem Schnarchen kein weiterer Schnarcher mehr detektiert wird, wird angenommen, dass der Schlafende mit Schnarchen aufgehört hat und die Schnarchphase zu Ende ist.

Um eine Gewöhnung an die Signale zu vermeiden, werden diese während einer Schnarchphase immer unangenehmer. Signal 1 ist noch relativ leise und in einem angenehmen Ton gehalten. Hierzu eignet sich z.B. ein Signal mit einer relativ tiefen Frequenz mit wenig Obertönen. Die folgenden Signale werden jedoch zunehmend wirksamer, indem z.B. ihr Fourierspektrum sich in höhere Frequenzbereiche verschiebt oder indem sie dissonanter werden. Die ersten Signale können auch völlig unterdrückt werden, so dass die Signalabgabe z.B. erst nach dem dritten Schnarchgeräusch beginnt.

In einer einfachsten Ausführung handelt es sich bei den akustischen Signalen um konstante Töne mit einer Grundfrequenz und harmonischen Frequenzkomponenten. Die Grundfrequenz ist beim ersten Signal relativ tief im Bereich einiger hundert Hertz und einem Bereich, in welchem die Empfindlichkeit des Ohrs noch nicht sehr hoch ist. Die Grundfrequenz steigt bei den folgenden Signalen an, bis sie mehrere Kilohertz erreicht.

Es ist jedoch auch denkbar, kompliziertere Signale zu verwenden. Insbesondere kann z.B. die Tonhöhe der unangenehmeren Signale in der Art einer Sirene zeitlich moduliert werden. Auch kann eine zunehmend nichtharmonische Frequenzverteilung verwendet werden.

Als Signale kommen auch kurze Tonsequenzen verschiedener Musikinstrumente oder menschlicher Stimmen (Worte) in Frage, welche z.B. in einem analogen EEPROM des Tongenerators gespeichert sind. Auch kann dem Schnarchenden eine Melodie vorgespielt werden, welche mit zunehmender Länge immer missklingender wird.

Auch Signale im Ultraschallbereich oder mit Ultraschallkomponenten sind denkbar.

Falls der Schnarcher lange nicht auf die Signale reagiert, beginnt die Steuerung verschiedenste Signale auszuprobieren. Hört das Schnarchen auf, so kann die Steuerung annehmen, dass das letzt gespielte Signal einen besonders starken Effekt hat. Dieses Signal merkt sich die Steuerung und verwendet es in einer nächsten Schnarchphase. Zeigt es sich jedoch, dass der Schnarcher auf ein eigentlich starkes, unangenehmes Signal nicht mehr reagiert, so kann angenommen werden, dass er sich daran gewöhnt hat. In diesem Fall wird das entsprechende Signal in Zukunft nicht oder nur zu Beginn einer Schnarchphase verwendet.

Hierzu weist die Steuerung einen Speicher auf, in welchem festgehalten wird, auf welche Signale der Schlafende stark reagiert (d.h. mit dem Schnarchen aufhört) und auf welche er keine Reaktion zeigt.

Wird das Gerät mit geeigneten Bedienungselementen versehen, so kann dieser Speicher auch manuell programmiert werden, indem z.B. dem wachen Benutzer verschiedene Signale vorgespielt werden und er zu jedem angibt, ob er es als angenehm oder unangenehm empfindet.

Da der Tongenerator z.B. 100 verschiedene Signale erzeugen kann, welche alle mit verschiedener Geschwindigkeit und Lautstärke abgegeben werden können, verfügt das Gerät über einen genügenden "Wortschatz", um immer wieder Signale zu finden, an welche sich der Schnarcher nicht gewöhnt hat. Soll noch ein grösserer Umfang an Signalen verwendet werden, so kann der Tongenerator rsp. dessen Signalspeicher auswechselbar oder sogar bespielbar gestaltet werden.

Vorzugsweise werden am Anfang einer Schnarchphase Signale ausgesendet, die auf den Schlafenden eine schwache Wirkung haben. Je länger die Schnarchphase dauert, desto wirkungsvollere Signale werden abgegeben. Dadurch wird erreicht, dass der auf den Schnarchenden ausgeübte Stimulus allmählich stärker wird. Sein Schlaf wird weniger tief und er hört mit dem Schnarchen auf, bevor er wach wird.

Mit dem beschriebenen Gerät kann eine Gewöhnung ausgeschlossen und ein Schnarchen sicher vermieden werden, ohne dass dazu übermassig laute Signale verwendet werden müssen.

## Patentansprüche

1. Vorrichtung zur Unterdrückung des Schnarchens eines Schläfers mit einem Detektionsgerät (1,2) zur Detektion des Schnarchens, mit einem Schallerzeuger (4,5, 6) und einer Steuerung (3), dadurch gekennzeichnet, dass mittels der Steuerung (3) und des Schallerzeugers (4,5,6) akustische Signale verschiedener spektraler Zusammensetzung erzeugbar sind, wobei die spektrale Zusammensetzung jedes Signals automatisch wählbar ist.

2. Vorrichtung nach Anspruch 1, dadurch gekennzeichnet, dass die spektrale Zusammensetzung der akustischen Signale in Abhängigkeit der Anzahl aufeinanderfolgender Schnarchgeräusche variierbar ist.

3. Vorrichtung nach Anspruch 2, dadurch gekennzeichnet, dass mit zunehmender Anzahl aufeinanderfolgender Schnarchgeräusche in den akustischen Signalen spektrale Komponenten höherer Frequenz stärker werden.

4. Vorrichtung nach einem der vorangehenden Ansprüche, dadurch gekennzeichnet, dass die spektrale Zusammensetzung der akustischen Signale mindestens teilweise nach einem Zufallsprinzip ausgewählt ist.

5. Vorrichtung nach einem der vorangehenden Ansprüche, dadurch gekennzeichnet, dass die spektrale Zusammensetzung mindestens einiger der Signale zeitlich variiert ist.

6. Vorrichtung nach einem der vorangehenden Ansprüche, dadurch gekennzeichnet, dass die spektrale Zusammensetzung jedes akustischen Signals im wesentlichen nur eine Grundfrequenz und harmonische Oberwellen umfasst.

7. Vorrichtung nach einem der vorangehenden Ansprüche, dadurch gekennzeichnet, dass die Steuerung (3) einen Speicher aufweist, in welchem festgehalten ist, auf welche Signale der Schläfer stark reagiert.

8. Vorrichtung nach Anspruch 7, dadurch gekennzeichnet, dass die Steuerung im Speicher festhält, mit welchen Signalen eine Schnarchphase beendet wurde.

9. Vorrichtung nach einem der vorangehenden Ansprüche, dadurch gekennzeichnet, dass sie ein Stirnband (9) aufweist, in welchem als Teil des Detektionsgeräts (1,2) ein Mikrofon (1) vorgesehen ist.

10. Vorrichtung nach einem der vorangehenden Ansprüche, dadurch gekennzeichnet, dass sie als Mikrofon ein Kehlkopfmikrofon umfasst.

11. Vorrichtung nach einem der vorangehenden Ansprüche, dadurch gekennzeichnet, dass der Schallerzeuger ein Stirnlautsprecher (6') ist und dass Mittel zur Halterung des Stirnlautsprechers (6') vorgesehen sind.

12. Vorrichtung nach einem der vorangehenden Ansprüche, dadurch gekennzeichnet, dass sie ein Stirnband (9) aufweist, in welchem der Schallerzeuger angeordnet ist.

13. Vorrichtung zur Unterdrückung des Schnarchens eines Schläfers mit einem Detektionsgerät (1,2) zur Detektion des Schnarchens, mit einem Schallerzeuger (4,5, 6) und einer Steuerung (3), dadurch gekennzeichnet, dass mittels der Steuerung (3) und des Schallerzeugers akustische Signale verschiedener spektraler Zusammensetzung und verschiedener Lautstärke erzeugbar sind, wobei die spektrale Zusammensetzung und Lautstärke automatisch änderbar ist.

## Claims

1. Device for the suppression of the snoring of a sleeper with a detection device (1,2) for detecting the snoring, a sound generator (4,5,6) and a control (3), characterised in that by means of the control (3) and the sound generator (4,5,6) acoustic signals of different spectral composition can be generated, wherein the spectral composition of each signal can be selected automatically.

2. Device of claim 1, characterised in that the spectral composition of the acoustic signals can be varied depending on the number of consecutive snoring sounds.

3. Device of claim 2, characterised in that in the acoustic signals spectral components of higher frequency become stronger with increasing number of consecutive snoring sounds.

4. Device of one of the preceding claims characterised in that spectral composition of the acoustic signals is selected at least partially according to a random principle.

5. Device of one of the preceding claims characterised in that the spectral composition of at least some of the signals is varied in time.

6. Device of one of the preceding claims characterised in that the spectral composition of each acoustic signal substantially comprises only one fundamental frequency and harmonics.

7. Device of one of the preceding claims characterised in that the control (3) comprises a memory, where it is registered to which signals the sleeper reacts strongly.

8. Device of claim 7, characterised in that the control registers in the memory the signals by means of which a snoring phase has been terminated.

9. Device of one of the preceding claims characterised in that it comprises a headband (9), in which a microphone (1) is provided as a part of the detection device (1,2).

10. Device of one of the preceding claims characterised in that it comprises a throat microphone as the microphone.

11. Device of one of the preceding claims characterised in that the sound generator is a forehead loudspeaker (6') and that means for holding the forehead loudspeaker (6') are provided.

12. Device of one of the preceding claims characterised in that it comprises a headband (9) in which the sound generator is arranged.

13. Device for suppressing the snoring of a sleeper with a detection device (1,2) for detecting the snoring, with a sound generator (4,5,6) and a control (3), characterised in that by means of the control (3) and the sound generator acoustic signals of different spectral composition and different volume can be generated, wherein the spectral composition and volume can be varied automatically.

## Revendications

1. Dispositif pour la suppression du ronflement d'un dormeur, avec un détecteur (1, 2) pour la détection du ronflement, un générateur acoustique (4, 5, 6) et un ensemble de commande (3), caractérisé en ce que celui-ci et le générateur acoustique (4, 5, 6) sont agencés pour produire des signaux acoustiques de composition spectrale variée, la composition spectrale de chaque signal pouvant être choisie automatiquement.

2. Dispositif selon la revendication 1, caractérisé en ce que la composition spectrale des signaux acoustiques est modifiable en fonction du nombre de ronflements successifs.

3. Dispositif selon la revendication 2, caractérisé en ce que les composantes spectrales de fréquence élevée dans les signaux acoustiques deviennent plus fortes lorsque le nombre de ronflements successifs augmente.

4. Dispositif selon une des revendications précédentes, caractérisé en ce que la composition spectrale des signaux acoustiques est au moins partiellement déterminée au hasard.

5. Dispositif selon une des revendications précédentes, caractérisé en ce que la composition spectrale d'au moins certains signaux varie dans le temps.

6. Dispositif selon une des revendications précédentes, caractérisé en ce que la composition spectrale de chaque signal acoustique ne comprend essentiellement qu'une fréquence fondamentale et des harmoniques supérieurs.

7. Dispositif selon une des revendications précédentes, caractérisé en ce que l'ensemble de commande (3) comporte une mémoire dans laquelle est stocké le type de signaux auquel le dormeur réagit fortement.

8. Dispositif selon la revendication 7, caractérisé en ce que l'ensemble de commande mémorise dans la mémoire quels sont les signaux terminant une phase de ronflement.

9. Dispositif selon une des revendications précédentes, caractérisé en ce qu'il comporte un serre-tête (9) dans lequel est disposé un micro (1) faisant partie du détecteur (1, 2).

10. Dispositif selon une des revendications précédentes, caractérisé en ce qu'il comporte un microphone de larynx faisant office de micro.

11. Dispositif selon une des revendications précédentes, caractérisé en ce que le générateur acoustique est un haut-parleur frontal (6'), et que le dispositif comporte des moyens pour supporter le haut-parleur frontal (6').

12. Dispositif selon une des revendications précédentes, caractérisé en ce qu'il comprend un serre-tête (9) dans lequel est agencé le générateur acoustique.

13. Dispositif pour la suppression du ronflement d'un dormeur, avec un détecteur (1, 2) pour la détection du ronflement, un générateur acoustique (4, 5, 6) et un ensemble de commande (3), caractérisé en ce que celui-ci et le générateur acoustique (4, 5, 6) sont agencés pour produire des signaux acoustiques de composition spectrale et d'intensité variées, la composition spectrale de chaque signal pouvant être modifiée automatiquement.
